# EUROPEAN PATENT APPLICATION

(11) **EP 2 154 131 A1**
(43) Date of publication of application: **17.02.2010**
(21) Application number: 08752016.9
(22) Date of filing: 24.04.2008
(51) Int. Cl.: C07D 213/74, A61K 31/423, A61K 31/4402, A61P 1/04, A61P 1/12, A61P 1/18, A61P 3/08, A61P 11/00, A61P 25/00, A61P 25/04, A61P 25/28, A61P 29/02, A61P 43/00, C07D 263/58

(54) **G PROTEIN-COUPLED RECEPTOR INHIBITOR AND PHARMACEUTICAL PRODUCT**

(30) Priority: 26.04.2007 JP 2007117458
(71) Applicant: Pharmafrontier, Co., Ltd., Kyoto 602-0841 (JP)
(72) Inventor: TSUJIMOTO, Gozo, Kyoto-shi Kyoto 606-8501 (JP); HIRASAWA, Akira, Kyoto-shi Kyoto 606-8501 (JP); MIYATA, Naoki, Nagoya-shi Aichi 467-0027 (JP); SUZUKI, Takayoshi, Nagoya-shi Aichi 467-0027 (JP); TAKAHARA, Yoshiyuki, Kyoto-shi Kyoto 602-0841 (JP)
(74) Representative: Hiebl, Inge Elisabeth
(86) International application number: PCT/JP2008/057917
(87) International publication number: WO 2008/139879

(57) **Abstract**

Disclosed are a substance and a pharmaceutical product inhibiting activation of a G protein-coupled receptor. Specifically disclosed is a G protein-coupled receptor inhibitor containing a specific compound represented by the following formula (1):

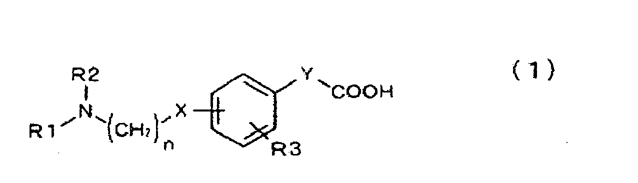

## Description

### TECHNICAL FIELD

The present invention relates to a G protein-coupled receptor inhibitor and a pharmaceutical product for treating digestive disorders, mental disorders, hypoglycemia, pulmonary diseases and the like.

### BACKGROUND ART

A physiologically active substance such as hormone or neurotransmitter, etc. can exhibit its activity via a specific receptor protein which is present in a cell membrane. Since a receptor protein typically performs intracellular signal transduction via activation of a guanidine triphosphate (GTP)-binding protein (i.e., GTP-binding protein), which is coupled to the receptor (herein below, sometimes abbreviated as "G protein"), it is generally referred to as a G protein-coupled receptor (protein). In addition, since the G protein receptor has a structure with seven transmembrane domains, it is also generally referred to as a seven-transmembrane receptor protein.

As one of the G protein-coupled receptors explained in the above, GPR120 which is present in an intestine, a lung, a brain, a pituitary gland, a fat cell and the like, is known, for example. Those which promote activation of an intracellular physiologically active substance such as G protein-coupled receptor and the like are called an agonist, while those inhibit the activation are called an antagonist. For example, according to Patent Document 1 and Non-patent Document 1, it is disclosed that intracellular Ca²⁺ increases when a ligand which activates GPR120 is added to a cell comprising GPR120, and subsequently, a peptide hormone such as cholecystokinin (CCK) or GLP-1 (glucagon-like polypeptide-1) is released.

Under the circumstance, it has been suggested to use a ligand, which is either an agonist or an antagonist, as an agent for treating various disorders. For example, according to Patent Document 1, it is described that, by administering a substance which promotes release of CCK, an eating disorder and symptoms of disorders that are associated therewith can be improved. According to Non-patent Document 1, it is described that, by administering a substance which can increase intracellular Ca²⁺ concentration, a secretion amount of GLP-1 or insulin can be increased, and therefore diabetes can be treated. Further, according to Patent Document 1, it is suggested that a substance which can promote release of CCK from a cell comprising GPR120 is used as an coordinative agent for promoting a digestion activity or as an agent for treating digestive disorders. Further, according to Non-patent Document 3, it is described that the ligand mentioned above can be used as a prophylactic and therapeutic agent for obesity or an agent for treating hyperphagia, based on suppression of appetite. According to Non-patent Document 4, it is suggested that the ligand mentioned above is used as a prophylactic and therapeutic agent for diabetes based on promotion of differentiation and proliferation of pancreatic β cells, in particular as an agent for promoting a therapeutic effect for transplant of the β cells or their precursor cells. According to Non-patent Document 5, it is suggested that the ligand mentioned above is used as an agent for promoting a therapeutic effect of neuron implant or neuron attachment treatment based on its activity of maintaining neural cell plasticity or cell survival, or as an agent for treating diseases that are derived from neuronal cell disorders such as Alzheimer's disease and the like. According to Non-patent Document 6, based on activity of normalizing intestinal movement, use of the ligand mentioned above as an agent for treating abnormal intestinal movement under a condition of a bowel disease is disclosed. According to Non-patent Document 7, it is disclosed that the ligand mentioned above is used as an agent for promoting an analgesic activity of analgesics such as morphine and the like or an agent for treating disorders derived from anxiety or stress, based on activity of lowering CCK concentration. According to Non-patent Document 8, based on activity of promoting secretion of a surfactant from lung, use of the ligand mentioned above as an agent for treating pulmonary diseases such as COPD (Chronic Obstructive Pulmonary Disease) and the like is disclosed.

Patent Document 1: Japanese Patent Application Laid-Open (JP-A) No. 2005-15358
Non-patent Document 1: Nature Medicine, 11(1), 90-94, 2005
Non-patent Document 2: Nutrition 2001; 17(3): 230-5
Non-patent Document 3: Physiol Behav. 2004; 83(4): 617-21
Non-patent Document 4: Diabetology, 2005; 48 (9) : 1700-13
Non-patent Document 5: Curr Drug Target CNS Neurol Disord 2002
Non-patent Document 6: Drug 2003; 63(12): 1785-97
Non-patent Document 7: Neurosci Biobehav Rev. 2005: 29(8): 1361-73
Non-patent Document 8: Endocrinology. 1998; 139(5): 2363-8

### DISCLOSURE OF THE INVENTION

### Problems to be Solved by the Invention

Meanwhile, although problems associated with a substance which inhibits a G protein-coupled receptor, i.e., an antagonist, have been pointed out, an idea of using it in a positive way is rarely suggested in the present moment.

Under the circumstances, inventors of the invention conducted a research regarding its use, and as a result, a finding related to the use of the antagonist of which use as a therapeutic agent has not been established was obtained.

Object of the invention is to establish a method for the beneficial use of a substance which inhibits a G protein-coupled receptor.

### Means for Solving the Problems

The G protein-coupled receptor inhibitor of the invention is an antagonist for G protein-coupled receptor (in particular, GPR120) which comprises as an effective component the specific compound represented by the following Formula (1) 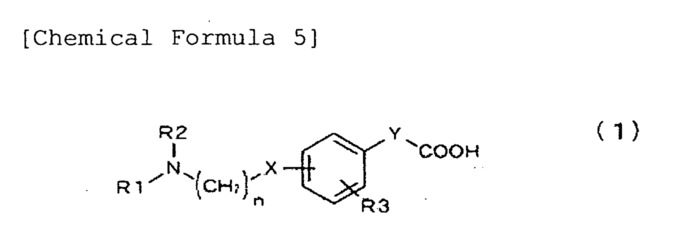 (wherein, R1 represents an aromatic hydrocarbon group, or an aromatic heterocyclic group, which may have a substituent group. R2 represents an alkyl group having 1 to 10 carbon atoms, an aromatic hydrocarbon group, or an aromatic heterocyclic group, which may have a substituent group. R3 represents a hydrogen (no substituent group), an alkyl group having 1 to 10 carbon atoms, an aromatic hydrocarbon group, an aromatic heterocyclic group, or an alkoxy group, which may have a substituent group. n is a natural number from 1 to 5. X represents an oxygen atom, a sulfur atom, or a nitrogen atom which may have a substituent group. Y represents an ethylene group which may have a substituent group, or a vinylene group which may have a substituent group. Two functional groups bonded to a benzene nucleus are at the meta or para position to each other).

Herein, GPR120 indicates one of the G protein-coupled receptors (herein below, it is sometimes abbreviated as GPCR) and corresponds to the polypeptides that are represented by the Sequence Listing Nos. 1 to 3.

As a result, use of a G protein-coupled receptor antagonist, which has been conventionally highlighted only in terms of the problems associated with life style-related diseases, for various purposes becomes possible. Specifically, under the purpose of treating a disorder which is caused by excessive activity of G protein-coupled receptors, a specific compound can be used for alleviating an activity of an agonist for the G protein-coupled receptor. Further, a specific compound can be used as an administration agent for producing in a cell or a living organism physiological states that are required for various experiments.

The specific compound of the invention may also be the one that is represented by the following Formula (2) 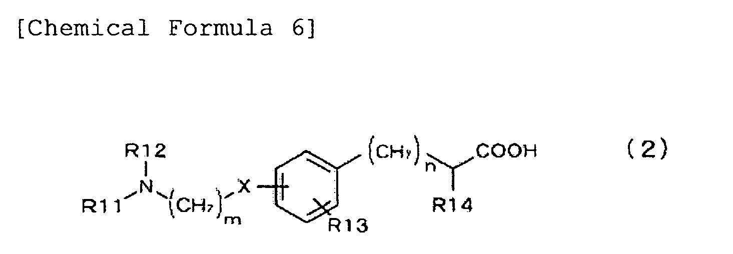 (wherein, R11 represents an aromatic hydrocarbon group, or an aromatic heterocyclic group, which may have a substituent group. R12 represents an alkyl group having 1 to 10 carbon atoms, an aromatic hydrocarbon group, or an aromatic heterocyclic group, which may have a substituent group. R13 represents a hydrogen (no substituent group), an alkyl group having 1 to 10 carbon atoms, an aromatic hydrocarbon group, an aromatic heterocyclic group, or an alkoxy group, which may have a substituent group. R14 represents a hydrogen (no substituent group), an alkyl group having 1 to 10 carbon atoms, an aromatic hydrocarbon group, an aromatic heterocyclic group, or an alkoxy group, which may have a substituent group. m and n are a natural number from 1 to 5. X represents an oxygen atom, a sulfur atom, or a nitrogen atom which may have a substituent group. Two functional groups bonded to the benzene nucleus are at the meta or para position to each other).
or it may also be the one that is represented by the following Formula (3) 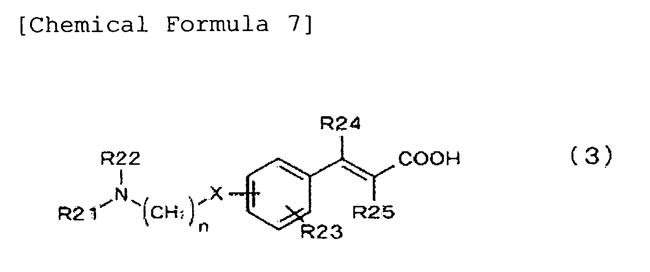 (wherein, R21 represents an aromatic hydrocarbon group, or an aromatic heterocyclic group, which may have a substituent group. R22 represents an alkyl group having 1 to 10 carbon atoms, an aromatic hydrocarbon group, or an aromatic heterocyclic group, which may have a substituent group. R23 represents a hydrogen (no substituent group), an alkyl group having 1 to 10 carbon atoms, an aromatic hydrocarbon group, an aromatic heterocyclic group, or an alkoxy group, which may have a substituent group. R24 represents a hydrogen (no substituent group), an alkyl group having 1 to 10 carbon atoms, an aromatic hydrocarbon group, an aromatic heterocyclic group, or an alkoxy group, which may have a substituent group. R25 represents a hydrogen (no substituent group), an alkyl group having 1 to 10 carbon atoms, an aromatic hydrocarbon group, an aromatic heterocyclic group, or an alkoxy group, which may have a substituent group. m and n are a natural number from 1 to 5. X represents an oxygen atom, a sulfur atom, or a nitrogen atom which may have a substituent group. Two functional groups bonded to the benzene nucleus are at the meta or para position to each other).

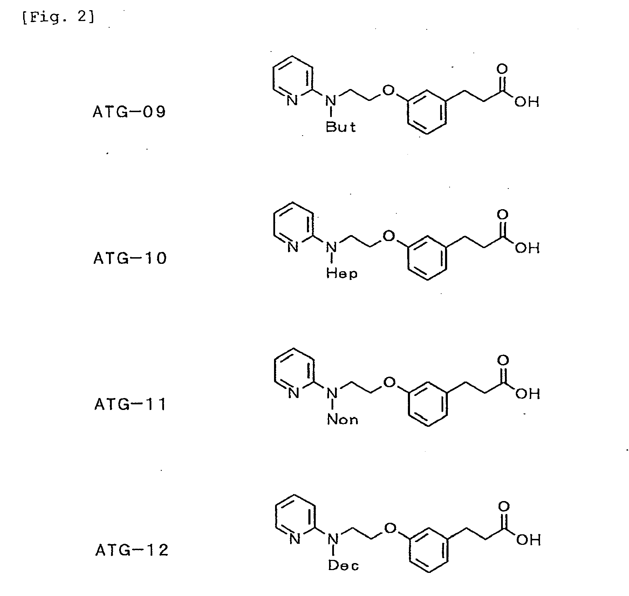

As for the compound that is represented by the above Formula (2), there are ATG-09 to ATG-15, ATG-17, ATG-18, ATG-20 and ATG-22 shown in Figs. 2 to 5.

As for the compound that is represented by the above Formula (3), there are ATG-16, ATG-19 and ATG-21 shown in Figs. 2 to 5.

With respect to the specific compound, it was confirmed that ATG-09, ATG-10, ATG-11, ATG-13, ATG-14, ATG-16, and ATG-19, etc. shown in Figs. 2 to 5 exhibit a significant antagonistic activity.

Conventionally, development of a pharmaceutical product for treating diabetes based on the use of an agonist for GPCR having fatty acids such as GPR120 or GPR40 as a ligand has been studied.

According to the present study, presence of a specific compound which blocks (inhibits) the function of a G protein-coupled receptor such as GPR120 and the like is confirmed. Specifically, it is based on a new mechanism in which a specific compound acts on intestinal hormone secretory cells and the like to inhibit the release of a mediator such as peptide hormone like cholecystokinin (CCK), chemokine and the like, and therefore inhibits disorders that are caused by the mediator. Thus, based on the new mechanism that is completely different from the one relating to conventional treatment of diabetes, etc., the specific compound of the invention exhibits a pharmaceutical effect that has not been known before.

The pharmaceutical product of the invention comprises as an effective component the G protein-coupled receptor inhibitor comprising the specific compounds described above, and therefore it can be used for treatment of various diseases. For example, based on an inhibitory action on increase in intracellular Ca²⁺ concentration which occurs in conjunction with the action of a G protein-coupled receptor (i.e., in an active state) in an intestine, a lung, a brain and the like having cells with GPR120, the agent can be used as a therapeutic agent for digestive disorders, a therapeutic agent for mental disorders, and a therapeutic agent for hypoglycemia and pulmonary diseases.

Further, by also using the pharmaceutical product of the invention as a combined preparation for treatment of various disorders using a ligand which promotes secretion of a physiologically active substance such as CCK, GLP-1 and the like, excessive increase in concentration of the physiologically active substance can be inhibited. As a result, it becomes possible to control concentration of the physiologically active substance in each cell of each organ at an appropriate level.

Disorders that can be treated according to the invention and efficacy are based on inhibition of increase in concentration of CCK present in blood or an organ to be treated and inhibition of an inflammatory mediator such as chemokine, etc. present in blood or an organ to be treated.

With respect to the disorders that can be treated based on decrease in CCK concentration, the followings can be mentioned, for example,
1) CCK promotes pancreatitis, thus pancreatitis can be treated by lowering CCK concentration
2) CCK causes a behavioral disorder due to anxiety and stress in a central system, thus the behavioral disorder can be treated by lowering CCK concentration
3) Hyperacidity is treated by inhibiting secretion of gastric acid via lowering CCK concentration
4) Analgesic effect is strengthened by lowering CCK concentration to antagonize analgesics such as morphine, etc.
5) Diarrhea such as infective diarrhea, stress diarrhea and the like is treated by lowering CCK concentration
6) Gastroesophageal reflux disease (GERD) is treated by lowering CCK concentration.
However, it is not limited to those described above. Instead, all of the treatments based on increased or decreased CCK concentration are included in the present invention.

Meanwhile, as a disorder to be treated according to inhibition of release of an inflammatory mediator such as chemokine, etc. via a G protein-coupled receptor like GPR120, etc., a bowel disease such as inflammatory bowel disease (IBD), irritable bowel syndrome (IBS) and the like, a neuronal disorder such as degenerative neuronal disease, immuno neuronal disease and the like, or a lung disease such as chronic obstructive pulmonary disease (COPD) and the like can be mentioned. However, it is not limited to these, and all of the treatments for a disorder relating to release of chemokine via a G protein-coupled receptor like GPR120, etc. are included in the invention.

The specific compound of the invention may directly act on a target organ or may inhibit release of an inflammatory mediator such as chemokine or a peptide hormone such as CCK, etc. via a G protein-coupled receptor like GPR120, etc. that is present in an intestine. Related applications are as follows.

1) An agent for treating pancreatitis
2) A tranquilizer
3) An agent for treating hyperacidity
4) An aid for analgesics like morphine, etc.
5) An anti-diarrheal agent
6) An agent for treating a bowel disease
7) An agent for treating a pulmonary disease, etc.

### Effect of the Invention

With the protein-coupled receptor inhibitor or the pharmaceutical product comprising it as an effective component according to the invention, an antagonist for a G protein-coupled receptor such as GPR120 and the like can be used effectively for various purposes.

### BRIEF DESCRIPTION OF THE DRAWINGS

Fig. 1 is a graph showing the amount of increase in Ca²⁺ concentration in cells comprising GPR120 according to α-Linolenic Acid (cis type) (α-LA (cis type)) stimulation, wherein the cells are either non-treated or treated for expression induction.
Fig. 2 is a diagram showing chemical formulae of ATG-09 to ATG-12.
Fig. 3 is a diagram showing chemical formulae of ATG-13 to ATG-18.
Fig. 4 is a diagram showing chemical formulae of ATG-19 to ATG-22.
Fig. 5 is a data which shows an inhibitory effect on activation when ATG-09 to ATG-12 are added to the cells comprising GPR120.
Fig. 6 is a data which shows an inhibitory effect on activation when ATG-13 to ATG-18 are added to the cells comprising GPR120.
Fig. 7 is a data which shows an inhibitory effect on activation when ATG-19 to ATG-22 are added to the cells comprising GPR120.
Fig. 8 is a diagram showing a process for preparation of ATG-19.

### BEST MODE FOR CARRYING OUT THE INVENTION

In the mode for carrying out the invention as described below, especially focusing on an antagonistic activity for GPR120, a GPCR inhibitor which comprises the specific compound of the invention as an effective component, and a pharmaceutical product comprising the GPCR inhibitor as an effective component will be explained.

GPR120 and the like is present in an intestine, a lung, a brain and the like, and it has been recognized that blocking GPR120 and the like by the specific compound of the invention has an effect of inhibiting increase in intracellular Ca²⁺ concentration, as it is disclosed in the Examples described below. Further, by inhibiting increase in Ca²⁺ concentration, release of a peptide hormone such as cholecystokinin (CCK) and GLP-1 is inhibited, for example. As a result, an effect of inhibiting diarrhea, etc. can be obtained, for example. Furthermore, since under a stress condition a hypersensitive reaction may occur in response to fatty acids or their derivatives derived from foods and it can be related to progress of a disease, the specific compound of the invention inhibiting GPR120 function is desirable as an agent for treating a bowel disorder such as IBS (irritable bowel syndrome) or IBD (inflammatory bowel disease) that is caused or worsened by stress load. Therefore, by having the specific compound of the invention as an inhibitor for GPR120 and the like, its use for disease treatment or internal reform can be achieved.

The G protein-coupled receptor inhibitor such as GPR120 and the like is formulated to be suitable for a therapeutically acceptable administration route including intravenous and oral administration. Specifically, the administration can be carried out according to the following aspects.

To prepare a formulation which is appropriate for injection, as a sterilized injectable solution or a medium for dispersion, a sterilized aqueous solution (water soluble) or a medium for dispersion, which can be prepared at the time of use, and sterilized powder (including freeze-dried protein, nucleic acids and the like) are included. As a carrier which is suitable for intravenous administration, physiological saline, bacteriostatic water, CREMOPHOR ELTM (BASF, Parsippany, N.J.), or phosphate buffered physiological saline (PBS) are included. When used as a formulation for injection, GPCR agonist, which is sterilized and administered via a syringe, should maintain its fluidity at sufficient level. Examples of a carrier which can be used include water, ethanol, polyol (glycerol, propylene glycol, and liquid polyethylene glycol and the like), and a solvent or a culture medium for dispersion comprising an appropriate mixture. For example, by using a coating agent such as lectin, etc., particle size that is required for a medium for dispersion can be obtained and also suitable fluidity can be maintained by having a surface active agent. Various types of an antibacterial agent and an antifungal agent, for example, paraben, chlorobutanol, phenol, ascorbic acid, timerosal and the like can be used for preventing contamination with microorganisms. Further, an agent which is useful for maintaining isotonicity, for example, polyalcohols such as sugar, mannitol, sorbitol and the like and sodium chloride can be included in the composition. For a composition which can delay adsorption, an agent such as aluminum monostearic acid, gelatin and the like is included.

Sterilized injectable solution is prepared by adding a required amount of a necessary active component alone or in combination with other components to an appropriate solvent and sterilizing the mixture. In general, a medium for dispersion can be prepared by introducing the active compound to a sterilized medium which comprises a basic culture medium for dispersion and other necessary components described above.
With respect to a method of preparing sterilized powder that is used for preparing sterilized injectable solution, vacuum drying and freeze drying are included by which powder comprising an active component and any one of desired components that are derived from a sterilized solution is prepared.

With respect to a formulation for oral administration, an inert dilution agent or a carrier which has no harm when it is absorbed by a human body is included. A formulation for oral administration is formed into a tablet as comprised in a gelatin capsule or pressurized. For a treatment with oral administration, an active compound is introduced with a vehicle and used in form including a tablet, a troche, and a capsule. Further, the formulation for oral administration can be prepared by using a carrier with fluidity. Still further, a binding agent and/or an adjuvant material and the like, which are pharmaceutically acceptable, can be also comprised.

A tablet, a pellet, a capsule, a troche and the similar products may comprise any one of the following components or a compound having a property similar to them. A vehicle such as microcrystalline cellulose and the like; a binding agent such as gum Arabic, tragacanth, gelatin and the like; an expanding agent such as starch, lactose, alginic acid, PRIMOGEL, cornstarch and the like; a lubricating agent such as magnesium stearate, STRROTES and the like; a lubricant such as colloidal silicon dioxide and the like; a sweetening agent such as sucrose, saccharine and the like, and; a flavoring agent such as peppermint, methyl salicylate, an orange flavor and the like.

With respect to a formulation for systemic administration, it can be administered transmucosally or transdermally. For transmucosal or transdermal administration, a penetrating agent which can be used for penetration into a barrier of a target is selected. As an agent for transmucosal penetration, a surface active agent, a bile acid salt, and fusidate derivatives are included. A nasal cavity spray or a suppository can be used for transmucosal administration. With respect to transmucosal administration, an active compound is formulated in an ointment, an unguent, a gel or a creme.

In addition, a pharmaceutical product which comprises the specific compound of the invention as an effective component can be also prepared as a suppository for delivery into a rectum (e.g., with a base material such as cocoa butter and other glycerides, etc.) or as a retentive enema form.

When the pharmaceutical product which comprises the specific compound of the invention as an effective component is formulated into a controlled release formulation, it can be prepared by using a carrier which is capable of preventing immediate removal in human body. For example, ethylene vinyl acetate and biodegradable and biocompatible polymer such as polyacid anhydride, polyglycolic acid, collagen, polyorthoester, polylactic acid and the like can be used. These materials can be obtained either from ALZA Corporation (Mountain View, CA) or NOVA Pharmaceuticals, Inc. (Lake Elsinore, CA), or can be easily prepared by a skilled person in the pertinent art. Further, a suspension comprising liposomes can be also used as a pharmaceutically acceptable carrier. Examples of useful liposomes are not limited, but include a lipid composition comprising phosphatidylcholine, cholesterol and PEG derivatized phosphatidylethanol (PEG-PE), and they are prepared by filtering through a filter with an appropriate pore size so as to be appropriate size for use and purified by a reverse phase evaporation method. For example, Fab' fragments of an antibody, etc. can be conjugated to a liposome based on a disulfide exchange reaction (Martin and Papahadjopoulos, 1982). For more detailed information regarding the method for preparation, descriptions in the literatures can be referenced (Eppstein et. al., 1985; Hwang et. al., 1980).

The administration amount of the pharmaceutical product which comprises the GPCR inhibitor of the invention as an effective component varies depending on a state of a patient (human) or an animal to be administered, an administration method, etc. for treatment or prevention of a certain disease. However, a skilled person in the pertinent art would have no difficulty optimizing them.

With respect to injection administration, about 0.1 µg to 500 mg is preferably administered per body weight kilogram of a patient per day, for example. In general, it can be administered once or several times per day with divided portions. Preferably, level of the administration amount is about 0.1 µg/kg to about 250 mg/kg per day, and more preferably about 0.5 µg/kg to about 100 mg/kg per day.

In case of oral administration, it is preferably provided as tablet form which comprises 1.0 to 1000 mg of an active component. Preferably, the administration amount of an effective active component relative to a patient (human) or an animal to be treated is between 0.01 and 100 mg/kg. The compound is administered according to an administration regimen in which 1 to 4 administrations are made per day. Preferably, it is administered once or twice per day.

In accordance with an activity of inhibiting release of CCK or GLP-1 based on concentration inhibition of intracellular ca²⁺ concentration by the GPCR inhibitor agonist of the invention, the following diseases can be treated, for example,
1. CCK promotes pancreatitis, thus pancreatitis can be treated by lowering CCK concentration
2. CCK promotes secretion of gastric acid, thus hyperacidity can be treated by inhibiting CCK concentration
3. CCK causes a behavioral disorder due to anxiety and stress in a central system, thus use as an agent for treating the behavioral disorder or as a tranquilizer can be achieved by inhibiting CCK concentration
4. CCK antagonizes analgesics such as morphine, etc, thus an analgesic effect can be strengthened by lowering CCK concentration
5. Diarrhea such as infective diarrhea and the like can be treated by inhibiting CCK concentration
6. Hypoglycemia can be treated by inhibiting GLP-1 concentration.
However, it is not limited to the diseases described above. Instead, treatment of various diseases based on decreasing CCK or GLP-1 concentration, or treatment of various diseases based on inhibited secretion of various physiologically active substances other than CCK or GLP-1 by inhibiting increase in Ca²⁺ concentration can be achieved.

### EXAMPLES

### Example 1

In the present example, a method of producing the specific compounds is explained with reference to Fig. 10, by having ATG-19
(E)-3-{3-[2-(2-nonylpyridinylamino)ethoxy]phenyl}acrylic acid as an example.

### (1) Process 1 : Production of N-nonylpyridin-2-amine (X)

2-Bromopyridine (0.9 mL) and n-nonyl amine (10 mL) were dissolved in toluene (10 mL) and tris(dibenzylideneacetone)dipalladium (0) (169 mg), 2,2'-bis(diphenylphosphino)-1,1'-binaphthyl (229 mg), and sodium tert-butoxide (1.2 g) were added thereto followed by stirring at 105°C for three days. To the reaction solution, ethyl acetate (100 mL) was added and the solution was washed with water (100 mL) . The organic layer was washed with water (100 mL) and saturated brine (50 mL) and dried over anhydrous sodium sulfate. After the filtration, concentration under reduced pressure was carried out. The residue was purified by silica gel flash column chromatography (developing solvent; n-hexane : ethyl acetate = 5:1) to obtain the title compound as a yellow solid (1.4 g, yield 70%). NMR data is given below. ¹H-NMR (CDCl₃, 500 MHz, d; ppm) 8.07 (1H, dd, J=4.9 Hz, 1.5 Hz), 7,41 (1H, ddd, J=8.5 Hz, 6.7 Hz, 1.8 Hz), 6.55 (1H, dd, J=6.7 Hz, 5.5 Hz), 6.37 (1H, d, J=8.2 Hz), 4.45 (1H, m), 3.24(2H, m), 1.65?1.59 (2H, m), 1.41?1.27 (12H, m), 0.88 (3H, t, J=6.9 Hz).

### (2) Process 2: Production of 3-(2-bromoethoxy)benzaldehyde (Y)

3-Hydroxy benzaldehyde (25.0 g) and 1,2-dibromoethane (52.8 mL) were dissolved in tetrahydrofuran (300 mL). Cesium carbonate (87. 6g) was added thereto and the mixture was stirred at 80°C for 120 hours. The reaction solution was poured over water and extracted with ethyl acetate (500 mL). The organic layer was washed with water (300 mL) and saturated brine (200 mL) and dried over anhydrous sodium sulfate. After the filtration, concentration under reduced pressure was carried out. The residue was purified by silica gel flash column chromatography (developing solvent; n-hexane : ethyl acetate = 5:1) to obtain the title compound (26.8 g, yield 57%). NMR data is given below.
¹H-NMR(CDCl₃, 500 MHz, d; ppm) 9.98 (1H, s), 7.51?7.46 (2H, m), 7.40(1H, t, J=1.4 Hz), 7.21 (1H, ddd, J=1.5, 2.7, 7.9 Hz), 4.36
(2H, t, J=6.1 Hz) 3.67 (2H, t, J=6.1 Hz).

### (3) Process 3 Production of ethyl (E)-3-(3-(2-bromoethoxy)phenyl)acrylate (Z)

Ethyldiethyl phosphonoacetate (0.8 mL) was dissolved in tetrahydrofuran (6mL), added with sodium hydride (180mg) under ice cooling and stirred for 1 hour under ice cooling. Further, the tetrahydrofuran solution (7 mL) comprising 3-(2-bromoethoxy)benzaldehyde (Y) (687 mg) that had been obtained from the above process was added thereto, and the mixture was stirred for 23 hours under ice cooling. The reaction solution was added to ice water and extracted with ethyl acetate (100 mL) . The organic layer was washed with water (50 mL) and saturated brine (50 mL) and dried over anhydrous sodium sulfate. After the filtration, concentration under reduced pressure was carried out. The residue was purified by silica gel flash column chromatography (developing solvent; n-hexane : ethyl acetate = 5:1) to obtain the title compound (848 mg, yield 95%). NMR data is given below.
¹H-NMR(CDCl₃, 500 MHz, d; ppm) 7.64 (1H, d, J=15.8 Hz),
7.31 (1H, t, J=7.9 Hz), 7.15 (1H, d, J=7.6 Hz), 7.06 (1H, t, J=2.1 Hz), 6.94 (1H, ddd, J=0.9, 2.4, 8.2 Hz), 6.42 (1H, d, J=16.1 Hz), 4.31 (2H, t, J=6.4 Hz), 4.27 (2H, q, J=7.3Hz), 3.65 (2H, t, J=6.1 Hz), 1.34 (3H, t, J=7 Hz).

### (4) Process 4: Production of ethyl (E)-3-{3-[2-(2-nonylpyridinylamino)ethoxy]phenyl}acrylate ester (W)

Ethyl (E)-3-(3-(2-bromoethoxy)phenyl)acrylate (Z) (502 mg) which had been obtained from the above process was dissolved in tetrahydrofuran (2 mL) and added with N-nonylpyridin-2-amine (X) (1.5 g) which had been obtained from the Process 1, triethylamine (0.67 mL) and potassium iodide (279 mg), followed by reflux under heating for 40 hours. To the reaction solution, ethyl acetate (100 mL) was added and washing was carried out with water (100 mL) and saturated brine (50 mL) followed by drying over anhydrous sodium sulfate. After the filtration, concentration under reduced pressure was carried out. The residue was purified by silica gel flash column chromatography (developing solvent; toluene : chloroform : ethyl acetate = 45:5:3) to obtain the title compound as a yellow oily matter (93 mg, yield 13%). NMR data is given below.
¹H-NMR(CDCl₃, 500 MHz, d; ppm) 8.16 (1H, ddd, J=1.2, 1.8, 4.8 Hz), 7,63(1H, d, J=15.8 Hz), 7.42 (1H, ddd, J=1.8, 7.0, 8.8 Hz), 7.01 (2H, m), 6.94 (1H, dd, J=1.8, 8.2 Hz), 6.52 (1H, dd, J=4.9, 7.3 Hz), 6.50 (1H, d, J=8.8 Hz), 6.41 (1H, d, J=16.1 Hz), 4.26 (2H, q, J=7.0 Hz), 4.21 (2H, t, J=6.1 Hz), 3.94 (2H, t, J=5.8 Hz), 3.46 (2H, t, J=7.9 Hz), 1.6571.61 (2H, m), 1.34 (3H, t, J=7.3 Hz), 1.35?1.25 (12H, m), 0.88 (3H, t, J=6.7 Hz).

### (5) Process 5 : Production of (E)-3-{3-[2-(2-nonylpyridinylamino)ethoxy]phenyl}acrylate (U, ATG-19) Ethyl

(E)-3-{3-[2-(2-nonylpyridinylamino)ethoxy]phenyl}acrylate ester (W) (84 mg) which had been obtained from the above process was dissolved in ethyl alcohol (1 mL) and tetrahydrofuran (1 mL) and added with 2N aqueous sodium hydroxide solution (0.69 mL), followed by stirring for 5 days at room temperature. To the reaction solution, 2N hydrochloric acid (0. 69 mL) was added. After the concentration, the residue was purified by silica gel flash column chromatography (developing solvent; chloroform : methanol = 19: 1) to obtain the title compound as a yellow oily matter (62 mg, yield 89%). NMR data is given below.
¹H-NMR(CDCl₃, 500 MHz, d; ppm) 8.17 (1H, dd, J=4. Hz, 1.8 Hz), 7.70 (1H, d, J=16.2 Hz), 7.44 (1H, ddd, J=8.8 Hz, 6.7 Hz, 2.1 Hz), 7.28 (1H, t, J=7.9 Hz), 7.10 (2H, m), 6.96 (1H, dd, J=8.5 Hz, 1.8 Hz), 6.54 (1H, dd, J=7.0 Hz, 5.2 Hz), 6.51 (1H, d, J=8.5 Hz), 6,41 (1H, d, J=15.8 Hz), 4.22 (2H, t, J=6.1 Hz), 3.95 (2H, t, J=6.1 Hz), 3.47 (2H, t, J=7.9 Hz), 1.66?1.61 (2H, m), 1.35?1.25 (12H, m), 0.88 (3H, t, J=7.0 Hz); HRMS Calcd for C₂₅H₃₄N₂O₃ 410.2569, Found
410.2567.
As shown in the above, Compound ATG-19 can be produced by various methods that are well known for generating each functional group. Similarly, although specific explanation regarding the preparation processes is not given, other compounds of the invention can be also produced by various methods that are well known for generating each functional group.

### Example 2

In the present example, as a G protein-coupled receptor, each construct of humanGPR120 (see, Sequence Listing 1) in which FLAG tag is attached to the N-terminal was prepared, and cells comprising GPR120 with a stable expression property were prepared. Then, by applying a certain treatment to the cells, the receptor was expressed and Ca²⁺ concentration was measured. It is also possible to use mouseGPR120 that is shown in Sequence Listing 2, instead of humanGPR120.

### 1. Measurement of intracellular Ca²⁺ concentration

(1) For the present experiment, a cell line which comprises GPR120 was used.
(2) Each cell was seeded to a 96 well clear bottom plate for cell culture of which surface had been treated with collagen to obtain the cell density of 2×10⁵ cells/well/50 µl. As a result, the cell plate was prepared.
(3) As a solution for seeding, FBS(-) medium comprising doxycyclin at the concentration of 10 µg/ml was used. By culturing the cells in this solution for 21 hours, a doxycyclin treatment and starvation were carried out simultaneously.

### 2. Loading of a Ca²⁺ ion sensitive fluorescent dye into the cells

(1) In order to load a calcium sensitive fluorescent dye into the cells, the reagent of Calcium Assay Kit, that is manufactured by Molecular Devices Corp., was used.
(2) The reagent was dissolved in FLIPR buffer (a solution which was prepared by adding 20 mM HEPES to Hanks' Balanced Salt Solution and then adjusting pH to 7.4) with the 2X concentration that is described in manuals for the Kit.
(3) Thus-prepared reagent solution of Calcium Assay Kit was added (50 µl/well) to the cell plate which had been kept for 21 hours after the seeding. Since the 50 µl/well medium had been used for the cell seeding, as a result of the present process, concentration of the reagent of Calcium Assay Kit in each well became the same as the predetermined concentration that is indicated in the manual.
(4) By keeping the cells to which the reagent solution of Calcium Assay Kit had been added in a dark place at room temperature for 1 hour, a calcium ion sensitive fluorescent dye that is comprised in the Kit reagent was loaded into the cells.

### 3. Ligand preparation

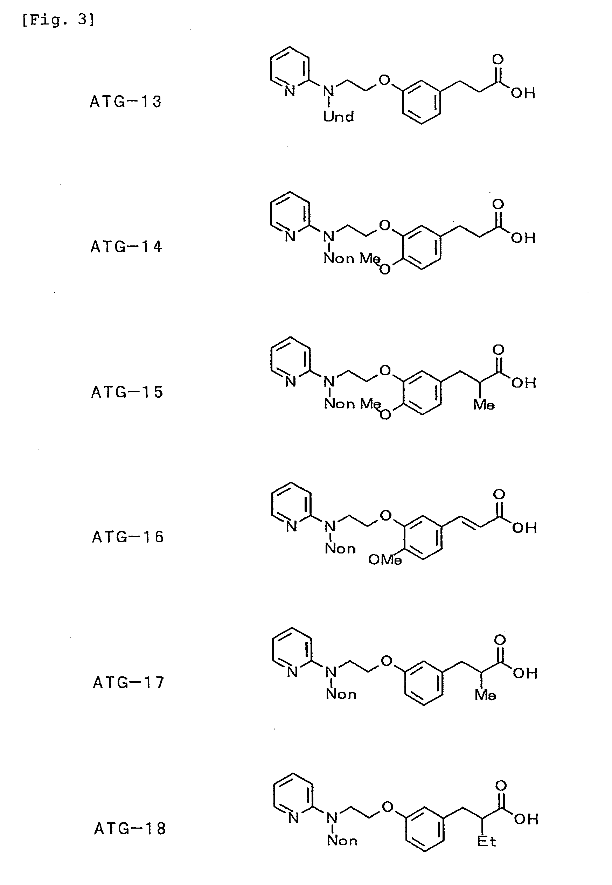

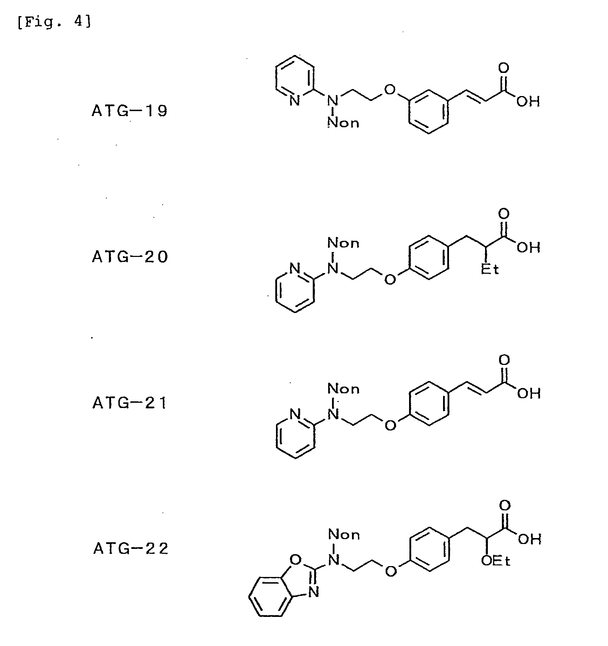

(1) As a compound to be tested (test compound), the compounds that are represented as specific compounds by ATG-09 to ATG-22 as shown in Figs. 2 to 4 were prepared. Fig. 2 shows chemical formulae of ATG-09 to ATG-12. Fig. 3 shows chemical formulae of ATG-13 to ATG-18. Fig. 4 shows chemical formulae of ATG-19 to ATG-22.
   The above compounds were dissolved in DMSO and diluted with the FLIPR buffer that has been explained before to give a solution comprising each compound at the concentration of 1.5×10⁻⁴ M and DMSO at the concentration of 1.5%.

(2) As a negative control, DMSO with the concentration same as the above was used. As a positive control, α-LA, which is a well known ligand for GPR120, was prepared in the same way as the each test compound.

(3) Measurement of increase in intracellular Ca²⁺ concentration
To the cells comprising GPR120, which had been either not treated (induction-) or treated for expression induction (induction+), 0.3% DMSO and various compounds with concentration from 3×10⁻⁸ to 1×10⁻⁴ M (0.3% DMSO) were added, and intracellular Ca²⁺ concentration was measured by FLIPR (manufactured by Molecular Devices Corp.). Peak values were detected and standardized (normalized) with reference to the peak value when 1×10⁻⁴ M α-LA (cis type) was added.

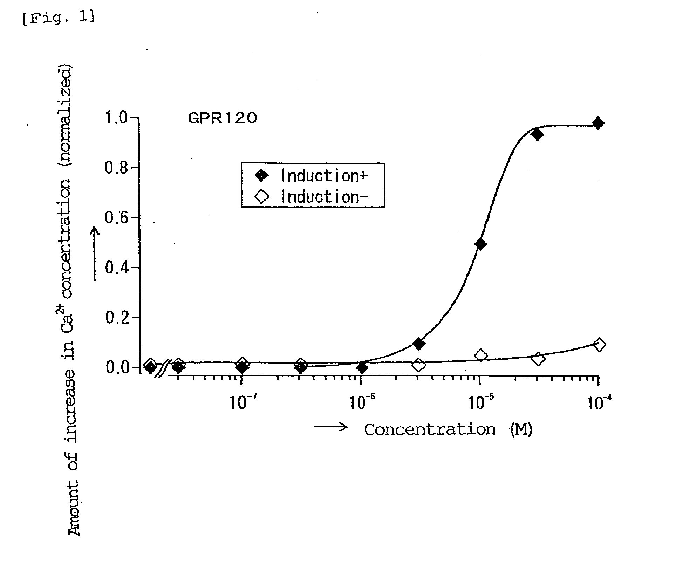

Fig. 1 corresponds to a data which shows an amount of increase in Ca²⁺ concentration in the non-treated cells (induction-) and the cells treated for expression induction (induction+) according to stimulation with α-LA (cis type α-Linolenic Acid), wherein the cells comprise GPR120. The horizontal axis of Fig. 1 indicates the addition amount of α-LA, and the vertical axis indicates the amount of increase in Ca²⁺ concentration. It was confirmed that, for all of the cells, increase in intracellular Ca²⁺ concentration is obtained by treatment for expression induction (induction+), and therefore the treatment for expression induction (induction+) had been appropriately carried out.

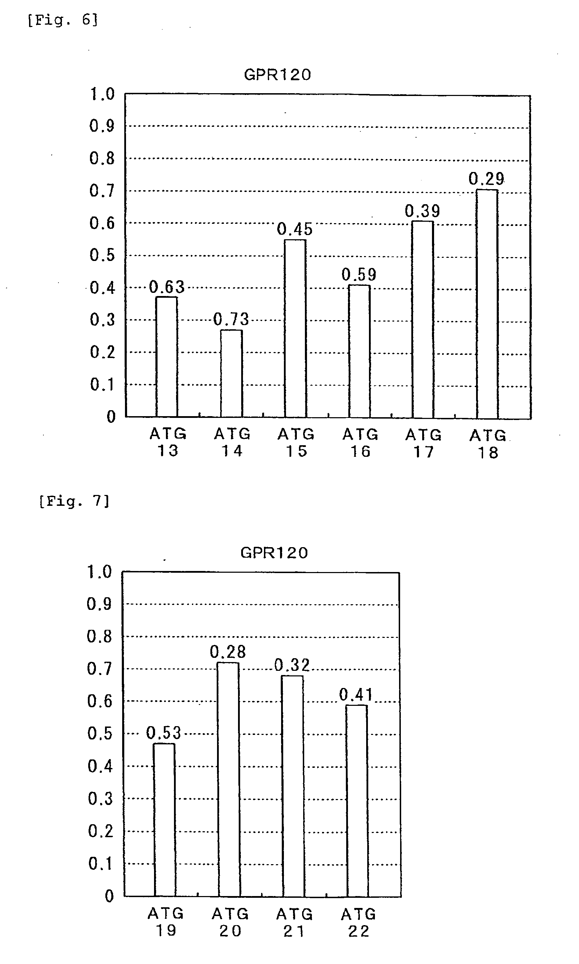

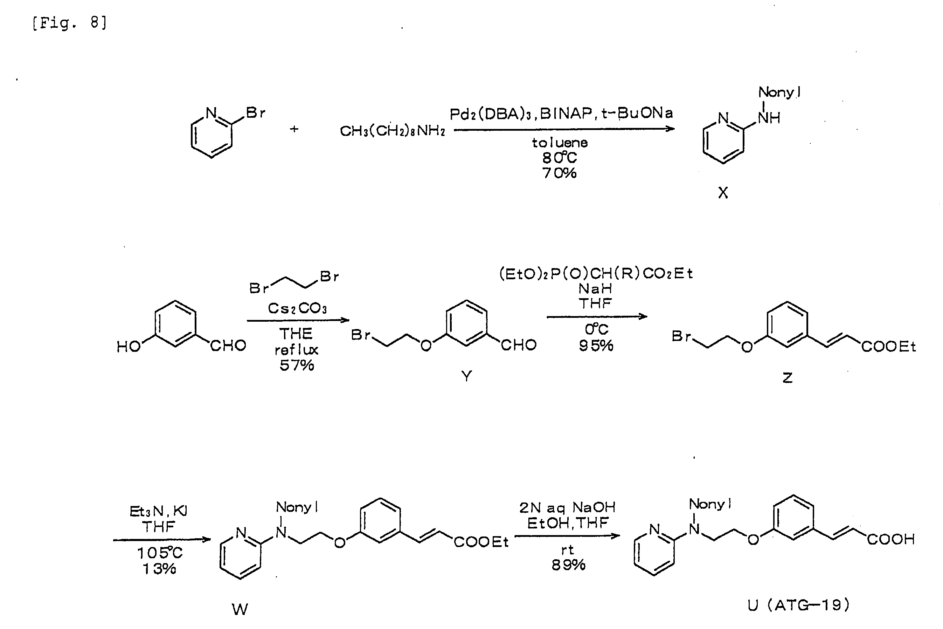

As explained in the above, intracellular Ca²⁺ concentration is related to the amount of a hormone as a physiologically active substance secreted from cells, and it is also confirmed to be an indicator which represents a physiologically active state of the cells. Therefore, from the data shown in Figs. 6 to 9 described below, the following aspects that are important for activation of each cell were confirmed.

(4) Confirmation of an inhibitory effect on cell activation
An inhibitory effect of a test compound on cell activation (i.e., an antagonistic activity) was evaluated by pre-treating the cells for ten minutes with a test compound at the same concentration as those for the evaluation of an agonistic activity (i.e., carrying out a treatment for activating the cells by expressing GPR120), and then stimulating the cells in an active state with α-LA at the same concentration to determine how much the increase in intracellular Ca²⁺ concentration is inhibited. Specifically, by using a multi-pipette, the test compound present in a plate for the pre-treatment was taken and added to each well of a cell plate that had been obtained after dye loading. Then, the cell plate and the compound plate in which α-LA (cis type) had been aliquoted were set in the FLIPR, which was then adjusted so that the addition of α-LA (cis type) is ten minutes after the addition of the test compound, followed by measurement of change in intracellular Ca²⁺ concentration over time as described above, before and after the stimulation by addition of α-LA (cis type). Further, from the present example, it was also confirmed that, in accordance with increase in intracellular Ca²⁺ concentration, secretion amount of GLP-1, a physiologically active substance secreted by the cells comprising GPR120, increased.

Further, after the completion of the measurement, peak value for the increase in fluorescence strength in the well which corresponds to the pre-treatment of each test compound was obtained. This value was normalized to the value for stimulation by α-LA when the pre-treatment was carried out only with DMSO. Consequently, an inhibitory effect of each test compound on the activation (i. e., an antagonistic activity) was evaluated.

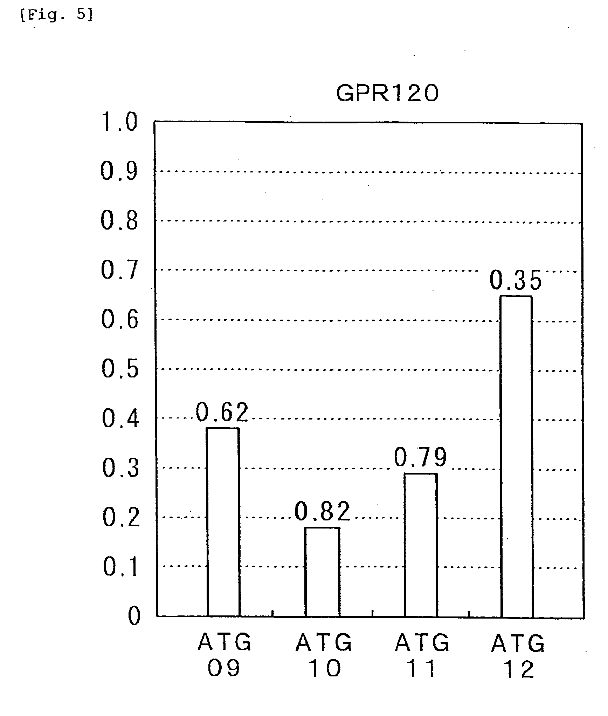

Fig. 5 is a data which shows an inhibitory effect on activation when ATG-09 to ATG-12 were added to the cells comprising GPR120. Fig. 6 is a data which shows an inhibitory effect on activation when ATG-13 to ATG-18 were added to the cells comprising GPR120. Fig. 7 is a data which shows an inhibitory effect on activation when ATG-19 to ATG-22 were added to the cells comprising GPR120. In Figs. 6 to 9, the vertical axis represents normalized Ca²⁺ concentration and the values indicate the inhibitory effect on activation (i.e., inhibitory level). In Figs. 6 to 9, the inhibitory effect on activation is bigger as the bar graph is shorter and the value is larger.

For example, according to the data shown in Fig. 5, Ca²⁺ concentration decreased to 0.18 (normalized value) by addition of ATG-10, when the Ca²⁺ concentration in an active state is set to 1. Further, it was significantly inhibited (reduced) to the level at which the activation of the cells comprising GPR120 was hardly observed. Therefore, the inhibitory effect of ATG-10 on the activation of the cells comprising GPR120 is 0.82 (normalized value). In addition, it is lowered to 0.38 (normalized value) by the addition of ATG-09, when the Ca²⁺ concentration in an active state is set to 1, and therefore the activation of the cells comprising GPR120 was significantly inhibited (reduced). The inhibitory effect of ATG-09 on activation of the cells comprising GPR120 is 0.62 (normalized value).

Meanwhile, by the addition of ATG-12, Ca²⁺ concentration decreased only to about 0.65 (normalized value), when the Ca²⁺ concentration in an active state is set to 1, but the activation of the cells comprising GPR120 was still somewhat inhibited (reduced). Therefore, although the inhibitory effect by ATG-12 on the activation of the cells comprising GPR120 is as small as 0.35 (normalized value), a constant antagonistic activity is obtained.

Based on the inhibitory effect by the specific compounds of the invention on the activation of the cells comprising GPR120, as shown in Figs. 5 to 7, it was confirmed that all the specific compounds have an antagonistic function for GPR120. In particular, ATG-09, ATG-10, ATG-11, ATG-13, ATG-14, ATG-16 and ATG-19 have an inhibitory level on activation of 0.5 (50%) or more, and therefore it was found that they have a significant antagonistic activity for a G protein receptor like GPR120, etc.

As it can be derived from the data shown in Figs. 5 to 7, by adding the specific compound of the invention to cells in an active state, the cell activity can be quantitatively inhibited. Although the active state is obtained by adding an agonist to a G protein-coupled receptor, for a condition in which GPR120 reaction is over-sensitized, for example, a condition in which GPR120 is over-expressed, the specific compound can be added as an antagonist to treat the condition. Therefore, the G protein-coupled receptor inhibitor which comprises as an effective component the specific compound of the invention can be used as a pharmaceutical product for treating diseases derived from an over-sensitive GPR120 reaction or as a nutritional supplement.

Conventionally, the activity of the specific compounds as an agonist or an antagonist for the G protein-coupled receptor was rarely known. However, according to the results of the present experiments, the antagonistic activity of the specific compounds (i.e., an inhibitor) for the G protein-coupled receptor, in particular GPR120, was confirmed. Further, with the pharmaceutical product comprising as an effective component the inhibitor for GPR120 of the invention, an effect as an agent for treating disorders or an agent for internal reform, etc. as described in the above can be obtained.

Further, although in the present example the intracellular Ca²⁺ concentration was measured as a way of quantitatively determining activation or inhibition of activation, detection of a physiologically active substance or a signal transducer including CCK, cytokine, ERK and the like can be also adopted.

Further, by using the pharmaceutical product of the invention comprising as an effective component the G protein-coupled receptor inhibitor which comprises the specific compound of the invention, treatment of various diseases can be achieved. Specifically, based on an inhibitory effect on increase in intracellular Ca²⁺ concentration which occurs in conjunction with the activation of a G protein-coupled receptor (i.e., an active state) present in an intestine, a lung or a brain which comprises cells with GPR120, the pharmaceutical product of the invention can be used as a therapeutic agent for digestive disorders, a therapeutic agent for mental disorders, a therapeutic agent for hypoglycemia, and a therapeutic agent for pulmonary diseases.

Further, as it is shown in the data of Figs. 6 to 9 and the literature "NATURE MEDICINE Vol. 11 No. 1 Jan. 2005 p. 90-94", when the G protein-coupled receptor inhibitor which comprises as an effective component the specific compound of the invention is administered to a cell comprising the corresponding receptor, the activation of the cell receptor is inhibited. As a result, a cell for screening in which an activity of a certain receptor is inhibited can be established. Still further, by adding a test compound to the cell and measuring an intestinal hormone in blood, for example GLP-1, CCK and the like, any ligand which is effective for recovery of the receptor function can be screened.

Similarly, as it is shown in the data of Figs. 6 to 9 and the literature "Am. J. Clin. Nutr. 2001; 73: p. 1019-1026", when the G protein-coupled receptor inhibitor which comprises as an effective component the specific compound of the invention is administered to an animal, the activation of the receptor that is present in cells of each organ of the animal is inhibited. As a result, an animal for screening in which an activity of a certain receptor is inhibited can be established. Still further, by adding a test compound to the animal and measuring an intestinal hormone in blood, for example GLP-1, CCK and the like, any ligand which is effective for recovery of the receptor function can be screened.

### INDUSTRIAL APPLICABILITY

The G protein-coupled receptor inhibitor of the invention can be used not only as a pharmaceutical product but also as a nutritional supplement.

SEQUENCE LISTING

<110>  PharmaFrontier, Co., Ltd.

<120>  G PROTEIN-COUPLED RECEPTOR INHIBITOR AND PHARMACEUTICAL PRODUCT

<130>  20651EP

<140>  EP 08 752 016.9
<141>  2008-04-24

<150>  JP 2007-117458
<151>  2007-04-26

<160>  3

<170>  PatentIn version 3.1

<210>  1
<211>  377
<212>  PRT
<213>  Homo sapiens

<400>  1

<210>  2
<211>  361
<212>  PRT
<213>  Homo sapiens

<400>  2

<210>  3
<211>  361
<212>  PRT
<213>  Mus musculus

<400>  3

## Claims

1. A G protein-coupled receptor inhibitor for inhibiting activity of a G protein-coupled receptor (GPCR) which comprises as an effective component the specific compound that is represented by the following Formula (1) 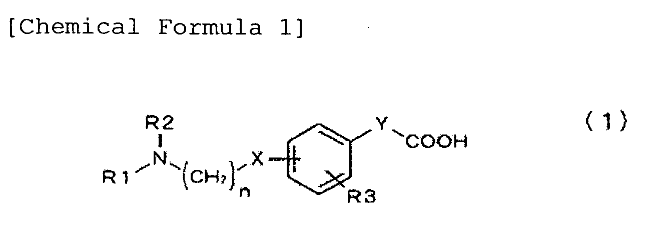 (wherein, R1 represents an aromatic hydrocarbon group, or an aromatic heterocyclic group, which may have a substituent group. R2 represents an alkyl group having 1 to 10 carbon atoms, an aromatic hydrocarbon group, or an aromatic heterocyclic group, which may have a substituent group. R3 represents a hydrogen (no substituent group), an alkyl group having 1 to 10 carbon atoms, an aromatic hydrocarbon group, an aromatic heterocyclic group, or an alkoxy group, which may have a substituent group. n is a natural number from 1 to 5. X represents an oxygen atom, a sulfur atom, or a nitrogen atom which may have a substituent group. Y represents an ethylene group which may have a substituent group, or a vinylene group which may have a substituent group. Two functional groups bonded to a benzene nucleus are at the meta or para position to each other).

2. The G protein-coupled receptor inhibitor according to Claim 1, in which the specific compound is a compound that is represented by the following Formula (2) 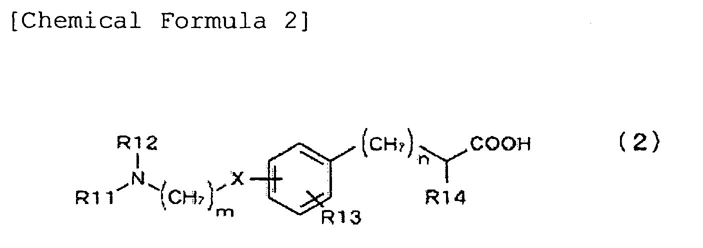 (wherein, R11 represents an aromatic hydrocarbon group, or an aromatic heterocyclic group, which may have a substituent group. R12 represents an alkyl group having 1 to 10 carbon atoms, an aromatic hydrocarbon group, or an aromatic heterocyclic group, which may have a substituent group. R13 represents a hydrogen (no substituent group), an alkyl group having 1 to 10 carbon atoms, an aromatic hydrocarbon group, an aromatic heterocyclic group, or an alkoxy group, which may have a substituent group. R14 represents a hydrogen (no substituent group), an alkyl group having 1 to 10 carbon atoms, an aromatic hydrocarbon group, an aromatic heterocyclic group, or an alkoxy group, which may have a substituent group. m and n are a natural number from 1 to 5. X represents an oxygen atom, a sulfur atom, or a nitrogen atom which may have a substituent group. Two functional groups bonded to the benzene nucleus are at the meta or para position to each other).

3. The G protein-coupled receptor inhibitor according to Claim 1, in which the specific compound is a compound that is represented by the following Formula (3) 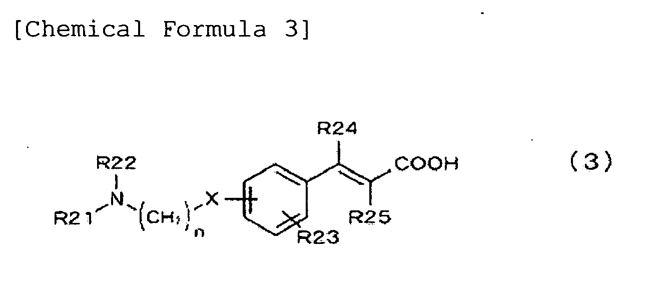 (wherein, R21 represents an aromatic hydrocarbon group, or an aromatic heterocyclic group, which may have a substituent group. R22 represents an alkyl group having 1 to 10 carbon atoms, an aromatic hydrocarbon group, or an aromatic heterocyclic group, which may have a substituent group. R23 represents a hydrogen (no substituent group), an alkyl group having 1 to 10 carbon atoms, an aromatic hydrocarbon group, an aromatic heterocyclic group, or an alkoxy group, which may have a substituent group. R24 represents a hydrogen (no substituent group), an alkyl group having 1 to 10 carbon atoms, an aromatic hydrocarbon group, an aromatic heterocyclic group, or an alkoxy group, which may have a substituent group. R25 represents a hydrogen (no substituent group), an alkyl group having 1 to 10 carbon atoms, an aromatic hydrocarbon group, an aromatic heterocyclic group, or an alkoxy group, which may have a substituent group. m and n are a natural number from 1 to 5. X represents an oxygen atom, a sulfur atom, or a nitrogen atom which may have a substituent group. Two functional groups bonded to the benzene nucleus are at the meta or para position to each other).

4. The G protein-coupled receptor inhibitor according to Claim 1, in which the specific compound is a compound that is selected fromATG-09, ATG-10, ATG-11, ATG-13, ATG-14, ATG-16 and ATG-19 as shown in Figs. 1 to 4.

5. The G protein-coupled receptor inhibitor according to any one of Claims 1 to 4, in which the G protein-coupled receptor is GPR120.

6. A pharmaceutical product comprising as an effective component a G protein-coupled receptor inhibitor which comprises as an effective component the specific compound that is represented by the following Formula (1) 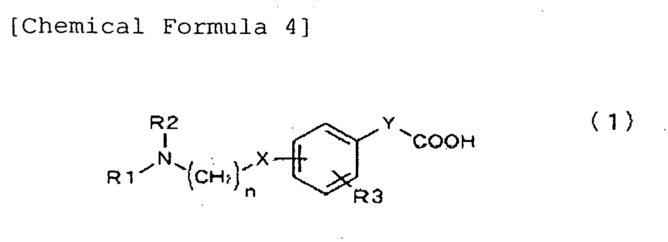 (wherein, R1 represents an aromatic hydrocarbon group, or an aromatic heterocyclic group, which may have a substituent group. R2 represents an alkyl group having 1 to 10 carbon atoms, an aromatic hydrocarbon group, or an aromatic heterocyclic group, which may have a substituent group. R3 represents an alkyl group having 1 to 10 carbon atoms, an aromatic hydrocarbon group, an aromatic heterocyclic group, or an alkoxy group, which may have a substituent group. n is a natural number from 1 to 5. X represents an oxygen atom, a sulfur atom, or a nitrogen atom which may have a substituent group. Y represents an ethylene group which may have a substituent group, or a vinylene group which may have a substituent group. Two functional groups bonded to a benzene nucleus are at the meta or para position to each other).

7. The pharmaceutical product according to Claim 6, in which the product functions as at least one therapeutic agent that is selected from a therapeutic agent for digestive disorders, a therapeutic agent for mental disorders, a therapeutic agent for hypoglycemia, and a therapeutic agent for pulmonary diseases.
